# EUROPEAN PATENT APPLICATION

(11) **EP 1 882 699 A1**
(43) Date of publication of application: **30.01.2008**
(21) Application number: 06015654.4
(22) Date of filing: 27.07.2006
(51) Int. Cl.: C07K 14/54

(54) **Regulation of interleukin-23**

(71) Applicant: Radeke, Heinfried, 60316 Frankfurt am Main (DE); Schleussner, Hans, 60528 Frankfurt (DE)
(72) Inventor: Radeke, Heinfried, 60316 Frankfurt am Main (DE)
(74) Representative: Köllner, Malte

(57) **Abstract**

A regulator of IL-23 is provided, which is based on a splice variant of the p19 subunit of IL-23. The invention comprises the regulator and substance, which influences the stimulating activity of the regulator, as active agents for the treatment of diseases, which are preferably connected with a disturbance of IL-23 activity.

SEQUENCE LISTING

<110> Prof. Dr. Radeke, Heinfried
      Dr. Dr. Schleussner, Hans

<120> Regulation of Interleukin-23

<130> RADPT06042EP

<160> 6

<170> PatentIn version 3.3

<210> 1
<211> 1358
<212> DNA
<213> Mus musculus


<220>
<221> CDS
<222> (113)..(703)
<223> Mus musculus p19 subunit of Interleukin-23

<300>
<301> Oppmann,B., Lesley,R., Blom,B., Timans,J.C., Xu,Y., Hunte,B.,
      Vega,F., Yu,N., Wang,J., Singh,K., Zonin,F., Vaisberg,E.,
      Churakova,T., Liu,M., Gorman,D., Wagner,J., Zurawski,S., Liu,Y.,
      Abrams,J.S., Moore,K.W., Rennick,D., de Waal-Malefyt,R.,
      Hannum,C., Bazan,J.F. and Kastelein,R.A.
<302> Novel p19 protein engages IL-12p40 to form a cytokine, IL-23,
      with biological activities similar as well as distinct from IL-12
<303> Immunity
<304> 13
<305> 5
<306> 715-725
<307> 2000-11-13
<308> AF301619
<309> 2000-12-04
<313> (1)..(1358)

<300>
<308> AF301619
<309> 2000-12-04
<313> (1)..(1358)

<400> 1
cgcttagaag tcggactaca gagttagact cagaaccaaa ggaggtggat agggggtcca      60

caggcctggt gcagatcaca gagccagcca gatctgagaa gcagggaaca ag atg ctg     118
                                                          Met Leu
                                                          1

gat tgc aga gca gta ata atg cta tgg ctg ttg ccc tgg gtc act cag       166
Asp Cys Arg Ala Val Ile Met Leu Trp Leu Leu Pro Trp Val Thr Gln
        5                   10                  15

ggc ctg gct gtg cct agg agt agc agt cct gac tgg gct cag tgc cag       214
Gly Leu Ala Val Pro Arg Ser Ser Ser Pro Asp Trp Ala Gln Cys Gln
    20                  25                  30

cag ctc tct cgg aat ctc tgc atg cta gcc tgg aac gca cat gca cca       262
Gln Leu Ser Arg Asn Leu Cys Met Leu Ala Trp Asn Ala His Ala Pro
35                  40                  45                  50

gcg gga cat atg aat cta cta aga gaa gaa gag gat gaa gag act aaa       310
Ala Gly His Met Asn Leu Leu Arg Glu Glu Glu Asp Glu Glu Thr Lys
                55                  60                  65

aat aat gtg ccc cgt atc cag tgt gaa gat ggt tgt gac cca caa gga 358
Asn Asn Val Pro Arg Ile Gln Cys Glu Asp Gly Cys Asp Pro Gln Gly
            70                  75                  80

ctc aag gac aac agc cag ttc tgc ttg caa agg atc cgc caa ggt ctg 406
Leu Lys Asp Asn Ser Gln Phe Cys Leu Gln Arg Ile Arg Gln Gly Leu
        85                  90                  95

gct ttt tat aag cac ctg ctt gac tct gac atc ttc aaa ggg gag cct 454
Ala Phe Tyr Lys His Leu Leu Asp Ser Asp Ile Phe Lys Gly Glu Pro
    100                 105                 110

gct cta ctc cct gat agc ccc atg gag caa ctt cac acc tcc cta cta 502
Ala Leu Leu Pro Asp Ser Pro Met Glu Gln Leu His Thr Ser Leu Leu
115                 120                 125                 130

gga ctc agc caa ctc ctc cag cca gag gat cac ccc cgg gag acc caa 550
Gly Leu Ser Gln Leu Leu Gln Pro Glu Asp His Pro Arg Glu Thr Gln
                135                 140                 145

cag atg ccc agc ctg agt tct agt cag cag tgg cag cgc ccc ctt ctc 598
Gln Met Pro Ser Leu Ser Ser Ser Gln Gln Trp Gln Arg Pro Leu Leu
            150                 155                 160

cgt tcc aag atc ctt cga agc ctc cag gcc ttt ttg gcc ata gct gcc 646
Arg Ser Lys Ile Leu Arg Ser Leu Gln Ala Phe Leu Ala Ile Ala Ala
        165                 170                 175

cgg gtc ttt gcc cac gga gca gca act ctg act gag ccc tta gtg cca 694
Arg Val Phe Ala His Gly Ala Ala Thr Leu Thr Glu Pro Leu Val Pro
    180                 185                 190

aca gct taa ggatgcccag gttcccatgg ctaccatgat aagactaatc 743
Thr Ala
195

tatcagccca gacatctacc agttaattaa cccattagga cttgtgctgt tcttgtttcg 803

tttgttttgc gtgaagggca aggacaccat tattaaagag aaaagaaaca aaccccagag 863

caggcagctg gctagagaaa ggagctggag aagaagaata aagtctcgag cccttggcct 923

tggaagcggg caagcagctg cgtggcctga ggggaagggg gcggtggcat cgagaaactg 983

tgagaaaacc cagagcatca gaaaaagtga gcccaggctt tggccattat ctgtaagaaa 1043

aacaagaaaa ggggaacatt atactttcct gggtggctca gggaaatgtg cagatgcaca 1103

gtactccaga cagcagctct gtacctgcct gctctgtccc tcagttctaa cagaatctag 1163

tcactaagaa ctaacaggac taccaatacg aactgacaaa tactaccact atgacctgtg 1223

acaaagctgc atatttatta agtgggaagg gaacttttga tattatttat ccttgtaaca 1283

gtatagatga tggttattta ttctatttat aaggaattat gtattttttt ttcaataaag 1343

atttatttat gtggc 1358

<210> 2
<211> 196
<212> PRT
<213> Mus musculus

<400> 2

Met Leu Asp Cys Arg Ala Val Ile Met Leu Trp Leu Leu Pro Trp Val

1                   5                   10                  15

Thr Gln Gly Leu Ala Val Pro Arg Ser Ser Ser Pro Asp Trp Ala Gln
            20                  25                  30

Cys Gln Gln Leu Ser Arg Asn Leu Cys Met Leu Ala Trp Asn Ala His
        35                  40                  45

Ala Pro Ala Gly His Met Asn Leu Leu Arg Glu Glu Glu Asp Glu Glu
    50                  55                  60

Thr Lys Asn Asn Val Pro Arg Ile Gln Cys Glu Asp Gly Cys Asp Pro
65                  70                  75                  80

Gln Gly Leu Lys Asp Asn Ser Gln Phe Cys Leu Gln Arg Ile Arg Gln
                85                  90                  95

Gly Leu Ala Phe Tyr Lys His Leu Leu Asp Ser Asp Ile Phe Lys Gly
            100                 105                 110

Glu Pro Ala Leu Leu Pro Asp Ser Pro Met Glu Gln Leu His Thr Ser
        115                 120                 125

Leu Leu Gly Leu Ser Gln Leu Leu Gln Pro Glu Asp His Pro Arg Glu
    130                 135                 140

Thr Gln Gln Met Pro Ser Leu Ser Ser Ser Gln Gln Trp Gln Arg Pro
145                 150                 155                 160

Leu Leu Arg Ser Lys Ile Leu Arg Ser Leu Gln Ala Phe Leu Ala Ile
                165                 170                 175

Ala Ala Arg Val Phe Ala His Gly Ala Ala Thr Leu Thr Glu Pro Leu
            180                 185                 190

Val Pro Thr Ala
        195

<210>  3
<211>  1025
<212>  DNA
<213>  Homo sapiens

<220>
<221>  CDS
<222>  (151)..(720)
<223>  Homo sapiens p19 subunit of Interleukin-23

<300>
<301>  Oppmann,B., Lesley,R., Blom,B., Timans,J.C., Xu,Y., Hunte,B.,
       Vega,F., Yu,N., Wang,J., Singh,K., Zonin,F., Vaisberg,E.,
       Churakova,T., Liu,M., Gorman,D., Wagner,J., Zurawski,S., Liu,Y.,
       Abrams,J.S., Moore,K.W., Rennick,D., de Waal-Malefyt,R.,
       Hannum,C., Bazan,J.F. and Kastelein,R.A.

<302>  Novel p19 protein engages IL-12p40 to form a cytokine, IL-23,
       with biological activities similar as well as distinct from IL-12
<303>  Immunity
<304>  13
<305>  5
<306>  715-725
<307>  2000-11-13
<308>  AF301620
<309>  2000-12-04
<313>  (1)..(1025)

<300>
<308>  AF301620
<309>  2000-12-04
<313>  (1)..(1025)

<400>  3
ccgaattcgg cacgagaaca actgagggaa ccaaaccaga gacgcgctga acagagagaa      60
tcaggctcaa agcaagtgga agtgggcaga gattccacca ggactggtgc aaggcgcaga     120
gccagccaga tttgagaaga aggcaaaaag atg ctg ggg agc aga gct gta atg      174
ctg ctg ttg ctg ctg ccc tgg aca gct cag ggc aga gct gtg cct ggg      222
ggc agc agc cct gcc tgg act cag tgc cag cag ctt tca cag aag ctc      270
tgc aca ctg gcc tgg agt gca cat cca cta gtg gga cac atg gat cta      318
aga gaa gag gga gat gaa gag act aca aat gat gtt ccc cat atc cag      366
tgt gga gat ggc tgt gac ccc caa gga ctc agg gac aac agt cag ttc      414
tgc ttg caa agg atc cac cag ggt ctg att ttt tat gag aag ctg cta      462
gga tcg gat att ttc aca ggg gag cct tct ctg ctc cct gat agc cct      510
gtg gcg cag ctt cat gcc tcc cta ctg ggc ctc agc caa ctc ctg cag      558
cct gag ggt cac cac tgg gag act cag cag att cca agc ctc agt ccc      606
agc cag cca tgg cag cgt ctc ctt ctc cgc ttc aaa atc ctt cgc agc      654
ctc cag gcc ttt gtg gct gta gcc gcc cgg gtc ttt gcc cat gga gca      702
gca acc ctg agt ccc taa aggcagcagc tcaaggatgg cactcagatc 750
tccatggccc agcaaggcca agataaatct accaccccag gcacctgtga gccaacaggt 810
taattagtcc attaatttta gtgggacctg catatgttga aaattaccaa tactgactga 870
catgtgatgc tgacctatga taaggttgag tatttattag atgggaaggg aaatttgggg 930
attatttatc ctcctgggga cagtttgggg aggattattt attgtattta tattgaatta 990
tgtacttttt tcaataaagt cttatttttg tggct 1025

<210> 4
<211> 189
<212> PRT
<213> Homo sapiens

<400> 4

<210>  5
<211>  1577
<212>  DNA
<213>  Mus musculus

<220>
<221>  CDS
<222>  (113)..(331)
<223>  Mus musculus splice variant of p19 subunit of Interleukin-23

<400>  5
cgcttagaag tcggactaca gagttagact cagaaccaaa ggaggtggat agggggtcca      60

caggcctggt gcagatcaca gagccagcca gatctgagaa gcagggaaca ag atg ctg     118
                                                          Met Leu
                                                          1

gat tgc aga gca gta ata atg cta tgg ctg ttg ccc tgg gtc act cag       166
Asp Cys Arg Ala Val Ile Met Leu Trp Leu Leu Pro Trp Val Thr Gln
        5                   10                  15

ggc ctg gct gtg cct agg agt agc agt cct gac tgg gct cag tgc cag       214
Gly Leu Ala Val Pro Arg Ser Ser Ser Pro Asp Trp Ala Gln Cys Gln
    20                  25                  30

cag ctc tct cgg aat ctc tgc atg cta gcc tgg aac gca cat gca cca       262
Gln Leu Ser Arg Asn Leu Cys Met Leu Ala Trp Asn Ala His Ala Pro
35                  40                  45                  50

gcg gga cat atg gta agt gtc agc tcc tgg gac cgc gca gaa aac ctt       310
Ala Gly His Met Val Ser Val Ser Ser Trp Asp Arg Ala Glu Asn Leu
                55                  60                  65

ccc agt cct cca agt gtg tag gtttaatgga agctgtggcc ccgggtggat          361
Pro Ser Pro Pro Ser Val
            70

ctggagggtt ggaagccatc gtggaatgag ataggacaga agactggggc ttctggaaga     421

gttgtgggcc ggcggttgag cggaatgcaa agcggtcacc tcgcctcact gttcccactc     481

cctccattac agaatctact aagagaagaa gaggatgaag agactaaaaa taatgtgccc     541

cgtatccagt gtgaagatgg ttgtgaccca caaggactca aggacaacag ccagttctgc     601

ttgcaaagga tccgccaagg tctggctttt tataagcacc tgcttgactc tgacatcttc     661

aaaggggagc ctgctctact ccctgatagc cccatggagc aacttcacac ctccctacta     721

ggactcagcc aactcctcca gccagaggat cacccccggg agacccaaca gatgcccagc     781

ctgagttcta gtcagcagtg gcagcgcccc cttctccgtt ccaagatcct tcgaagcctc     841

caggcctttt tggccatagc tgcccgggtc tttgcccacg gagcagcaac tctgactgag     901

cccttagtgc caacagctta aggatgccca ggttcccatg gctaccatga taagactaat     961

ctatcagccc agacatctac cagttaatta acccattagg acttgtgctg ttcttgtttc    1021

gtttgttttg cgtgaagggc aaggacacca ttattaaaga gaaaagaaac aaaccccaga    1081

gcaggcagct ggctagagaa aggagctgga gaagaagaat aaagtctcga gcccttggcc    1141

ttggaagcgg gcaagcagct gcgtggcctg aggggaaggg ggcggtggca tcgagaaact    1201

gtgagaaaac ccagagcatc agaaaaagtg agcccaggct ttggccatta tctgtaagaa 1261

aaacaagaaa aggggaacat tatactttcc tgggtggctc agggaaatgt gcagatgcac 1321

agtactccag acagcagctc tgtacctgcc tgctctgtcc ctcagttcta acagaatcta 1381

gtcactaaga actaacagga ctaccaatac gaactgacaa atactaccac tatgacctgt 1441

gacaaagctg catatttatt aagtgggaag ggaacttttg atattattta tccttgtaac 1501

agtatagatg atggttattt attctattta taaggaatta tgtatttttt tttcaataaa 1561

gatttattta tgtggc 1577

<210> 6
<211> 72
<212> PRT
<213> Mus musculus

<400> 6

Met Leu Asp Cys Arg Ala Val Ile Met Leu Trp Leu Leu Pro Trp Val
1               5                   10                  15

Thr Gln Gly Leu Ala Val Pro Arg Ser Ser Ser Pro Asp Trp Ala Gln
            20                  25                  30

Cys Gln Gln Leu Ser Arg Asn Leu Cys Met Leu Ala Trp Asn Ala His
        35                  40                  45

Ala Pro Ala Gly His Met Val Ser Val Ser Ser Trp Asp Arg Ala Glu
    50                  55                  60

Asn Leu Pro Ser Pro Pro Ser Val
65                  70

## Description

The invention relates to a regulator of interleukin-23 (IL-23) and uses of this regulator.

Interleukin-23 (IL-23) is a heterodimeric cytokine composed of the p40 and p19 subunit the first of which also constituting part of the IL-12 heterodimer. IL-23 induces a unique T helper cell subset to produce IL-17, which plays a critical and IL-12/IFNγ-independent role in autoimmunity. Plasmacytoid dendritic cells (pDCs) as opposed to myeloid DCs (mDC) and the closely related epidermal Langerhans cells (LC) exhibit a specific and broader range of pro-inflammatory cytokine secretion, with type-1 interferons representing a major difference to classical mDCs and LC. In this invention it will be shown that upon treatment with a selection of Toll-like receptor (TLR) ligands for TLR3, -4, -7 and -9, only mDCs and LC but not pDCs secreted IL-23. While pDC produced both mRNA and protein of the p40 subunit, the lack of bioactive heterodimeric IL-23 protein release was related to the fact that in these cells only the p19 mRNA was expressed which was not translated into protein. In addition to these differential findings in both DC subsets a novel p19 splice variant was identified. This analysis of transcriptional and/or post-transcriptional regulation of the IL-23 subunits p40 and p19 helps to understand the complex regulation of heterodimeric cytokines and the overlapping but distinct functions of IL-12 and IL-23. It shows that a coordinated adaptive immune response based on a finely tuned contribution of these cytokines by different mouse DC subsets is established.

Interactions between dendritic cell (DC) subsets are important for a coordinated immune response upon viral or bacterial infections. Plasmacytoid DCs (pDCs) activate natural killer cells, B cells, T cells and other DCs from the myeloid lineage via massive and fast production of Interferon-alpha (IFN-α) upon stimulation. Even though mouse pDCs have the capacity to produce both IFN-α and IL-12, mDCs are specialized in producing IL-12 but not type I IFNs (Liu, 2005). Whereas conditions for production of IFN-α and IL-12 by distinct DC subsets are well established, little is known about the differential production of the IL-12 relative IL-23. IL-23 is a heterodimeric cytokine composed of the p40 and p19 subunits. While the p19 subunit is unique for IL-23 p40 is shared with IL-12 (Oppmann et al., 2000). The receptor used by IL-23 is formed by association of IL-12Rβ1 and IL-23R (Parham et al., 2002;Oppmann et al., 2000). Thus, together with IL-6, IL-12, and IL-27, IL-23 belongs to a group of cytokines with promiscuous use of receptor and ligand components. The main sources of IL-23 are macrophages and dendritic cells (DCs). Upon stimulation with Toll-like receptor (TLRs) ligands such as LPS (Middleton et al., 2006) or peptidoglycan (Smits et al., 2004) or interaction with T cells (CD40/CD40L interaction) (Krajina et al., 2003; Oppmann et al., 2000; Smits et al., 2004; Jefford et al., 2003; Langrish et al., 2004) antigen presenting cells (APCs) can produce IL-23. In addition, prostaglandin E2 induces p19 and p40, but not p35 expression in immature DCs (Sheibanie et al., 2004).

Due to the most recently developed concept IL-23 seems to have a dual function during an immune response and supplementary role compared to the dominant function of IL-12 (McKenzie et al., 2006): within the first hours secreted by resident APC/DC it acts in a auto- and paracrine manner to induce nitric oxide (Langrish et al., 2004) and IL-17. IL-17 in turn will activate local cells to amplify the innate reaction by expression of monokines and mainly neutrophil specific chemokines (Alber et al., 2006; Kleinschek et al., 2006). Later, and especially when a dominant Th1 or Th2 activity is lacking IL-23 secreting APCs initiate Th17 from naive T cells (Wynn, 2005) and may enhance IFNγ secretion of memory T lymphocytes (McKenzie et al., 2006). Stat1 activators like type 1 and IFNs down regulate the IL-23/IL-17 axis, while on the other hand they participate in a stimulatory feedback loop for the IL-12/IFNγ or Th1 immunity (Wynn, 2005; Harrington et al., 2005).

To date, the role of IL-23 in protection against infections is poorly understood. Studies comparing p35- and p40-deficient animals that are devoid of IL-12 or IL-12 and IL-23, respectively, made evident that p40-dependent and p35-independent mechanisms of resistance to several pathogens can be discriminated. Clearance of the intracellular bacterium *Francisella turalensis* LVS was dependent on the presence of the p40 subunit but not on IL-12p70 (Elkins et al., 2002) indicating that either p40 alone or IL-23 might play a critical role. The observation that administration of IL-23 to p40-deficient mice resuited in increased resistance to toxoplasmosis indicated that IL-23 provides a mechanism of resistance in the absence of IL-12p70 (Lieberman et al., 2004). Studies using the gram-negative bacterium *Klebsiella pneumoniae* provided further insights into the role of IL-23 during infection: previous reports evidenced that IL-17 has a central role in the resistance against *K. pneumoniae* (Ye et al., 2001). *Klebsiella pneumoniae-*pulsed DCs were capable of producing IL-23, which induces production of IL-17 by T helper cells (Happel et al., 2003). In line with these observations, p19-deficient mice infected with *Klebsiella pneumoniae* cannot produce IL-17 and show an increased susceptibility to infection (Hunter, 2005).

More is known about the role of IL-23 in autoimmunity and chronic inflammation. Experiments with p40-deficent mice or p40 neutralizing antibodies revealed that cytokines consistent of p40 are essential for the development of T cell mediated autoimmune diseases like experimental allergic encephalomyelitis (EAE) (Leonard et al., 1995) or collagen-induced arthritis (CIA) (Malfait et al., 1998; McIntyre et al., 1996). While p19-deficient mice are resistant to the induction of CIA, p35-deficient mice showed an increase of disease severity. Mice lacking p40 subunit are protected against EAE, whereas p35-deficient mice develop a more severe pathology than wild-type mice (Becher et al., 2002; Gran et al., 2002; Cua et al., 2003). In contrast, p19-deficent mice are resistant to EAE (Cua et al., 2003). This indicates that there is a role of IL-23 but not of IL-12 in the development of EAE. Aberrant expression of p19 induced multi organ inflammation (Wiekowski et al., 2001) and deregulation of IL-23 production by basal keratinocytes that may lead to the development of eczematous skin disease (Kopp et al., 2003). Moreover, p19-deficient mice showed reduced T cell mediated delayed-type hypersensitivity responses, which is in line with a reduced capacity to produce IL-17 (Ghilardi et al., 2004).

Comparative studies using p40- or p35-deficient mice indicated that there is a need in understanding the regulation of the expression of the different subunits to distinguish the overlapping but distinct functions of IL-12 and IL-23. In this invention detailed analysis of transcriptional and/or post-transcriptional regulation of the IL-23 subunits p40 and p19 by different mouse dendritic cell subsets is provided. Interestingly, plasmacytoid DCs (pDCs) which are very potent producers of pro-inflammatory cytokines upon treatment with a variety of different stimuli were incapable of secreting IL-23. In contrast, classical myeloid DCs and closely related tissue resident Langerhans cells turned out to be the main source of IL-23. Furthermore, a novel p19 splice variant could be detected in both pDC and mDC.

Changes in interleukin-23 (IL-23) expression, activity and tissue distribution have been described in a variety of disorders ranging e.g. from Psoriasis, Multiple Sclerosis, Rheumatoid Arthritis, Diabetes, Morbus Crohn, Ulcerative Colitis, Transplantation, to different types of Cancer.

Because IL-23 plays a crucial role in widespread diseases like colitis, rheumatism and psoriasis, there is a special interest in the regulatory mechanisms concerning the activity of IL-23. The detailed knowledge of the regulation mechanisms would provide a possibility to influence the activity of IL-23, especially in pathological situations. Thus, the invention has the object to provide suited targets for influencing the activity of this crucial cytokine and to provide active agents for the treatment of diseases, which are connected with a disturbance of IL-23 activity.

This object is solved by a regulator of IL-23 as described in claim 1. Preferred embodiments of this regulator are described in the dependent claims 2 and 3. The corresponding nucleotide sequences and amino acid sequences are claimed in the claims 4 - 7. In claims 8 - 12 an active agent for the treatment of diseases is described. Claims 13 - 16 relate to the use of said active agent a method for the treatment of diseases and a pharmaceutical composition. Claims 17 - 22 concern the use of a regulator for diagnosis of diseases and a corresponding kit. Claims 23 - 25 describe a method for searching substances, and the resulting active agent. The wording of all claims is hereby made to the content of the specification by reference.

The invention comprises a regulator of IL-23, which is based on a splice variant of the p19 subunit of IL-23. In comparison with the complete subunit (gene bank entry AF301619) this regulator contains an additional exon which has not been spliced out. This additional exon is 219 bp long and encodes for a stop codon after 18 novel amino acids leading to a shortened protein. This inventive regulator is of high interest, especially with respect a novel regulatory step at translational or post-translational level. A truncated p19 protein worked as a molecular block for an effective assembly of the IL-23 heterodimer. Thereby the bioactive IL-23 heterodimer can not exert its function in several diseases.

According to the invention, the "regulator" is either the splice variant and/or the subsequent shortened protein or a factor which regulates the splicing of the precursor-mRNA to either the mRNA leading to a bioactive p19 component of the the p19/p40 heterodimer of bioactive IL-23, or to the splice variant of the precursor p19 mRNA, which contains an additional exon leading to a longer mRNA variant but (due to a Stop Codon) to a p19 protein variant with a reduced size. The latter in addition to the "splice regulator" may be an "regulator" in itself leading to the blockade of the assembly of p40 with the bioactive long version of the p19 protein.

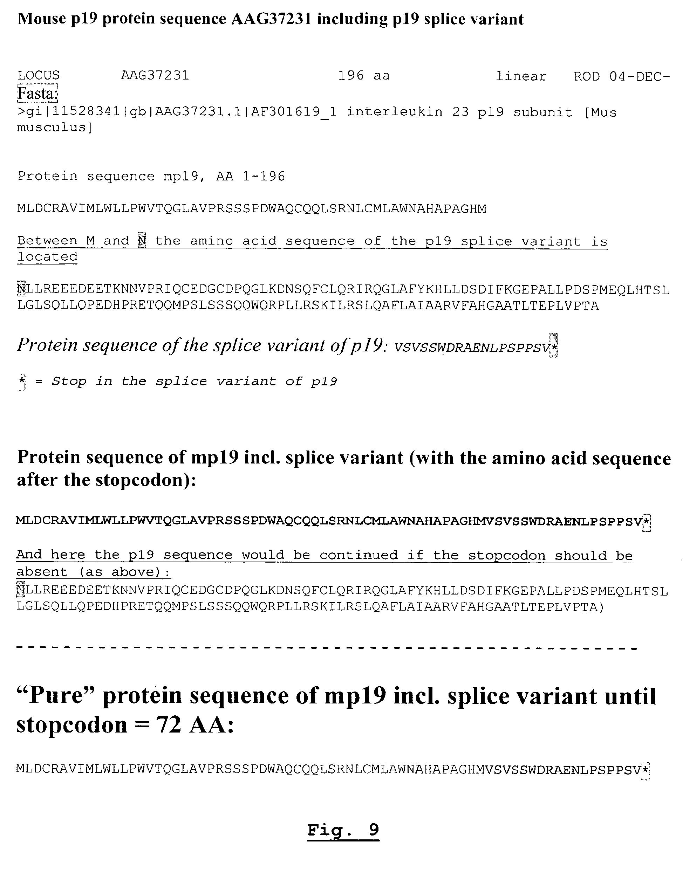

In a preferred embodiment of the invention the regulator is characterized by an amino acid sequence at least 70% and preferably at least 80%, especially at least 90% identical to the amino acid sequence according to figure 9 (SEQ ID NO 6). Preferably these amino acid sequences represent the amino acid sequence of a splice variant of the p19 subunit of IL-23.

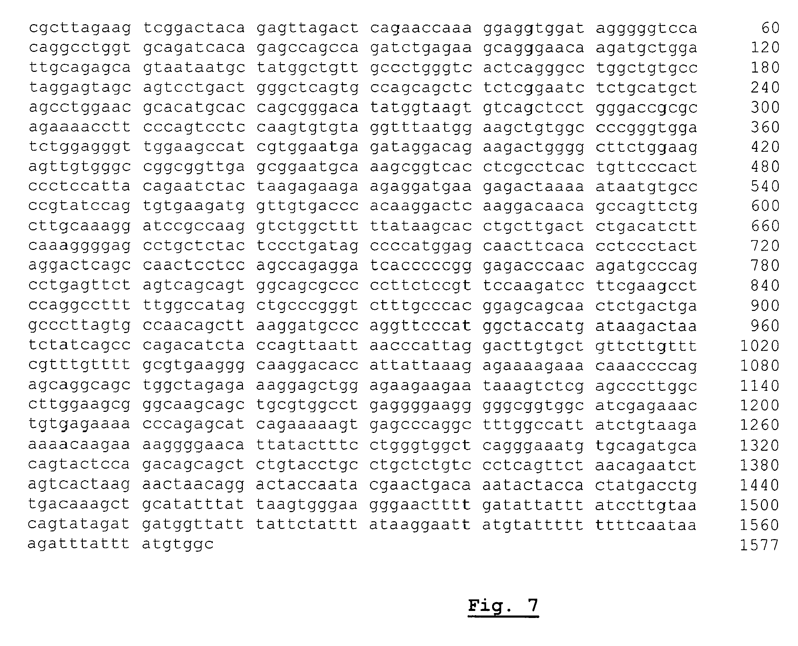

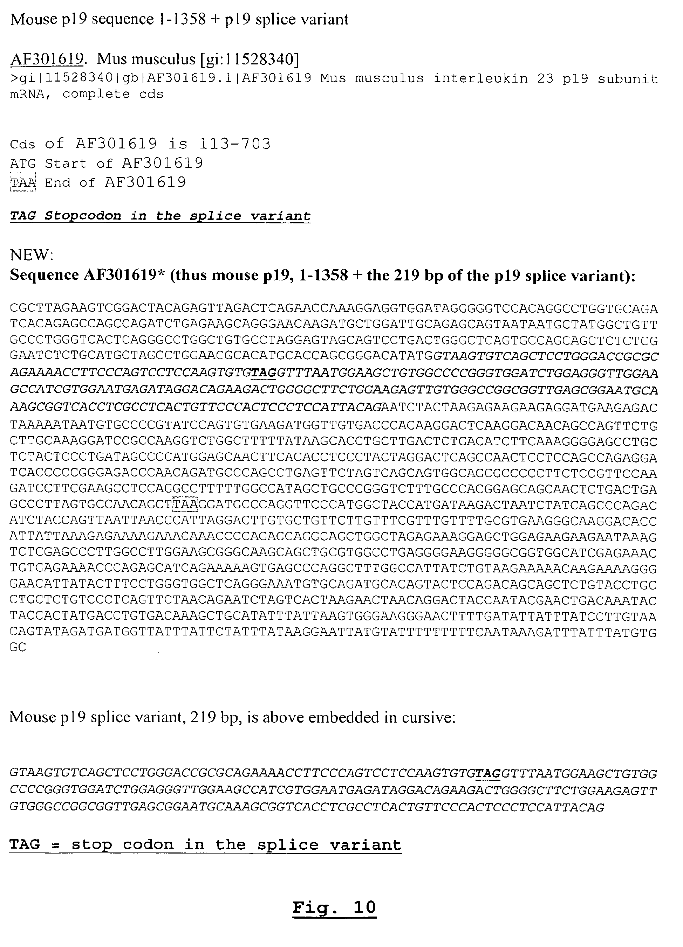

In another embodiment the inventive regulator is coded by a nucleotide sequence at least 70% and preferably at least 80%, especially at least 90% identical to the nucleotide sequence according to figure 7 (SEQ ID NO 5), and/or figure 10 (SEQ ID NO 5). Preferably these nucleotide sequences represent the nucleotide sequence of a splice variant of the p19 subunit of IL-23.

Furthermore, the invention comprises a nucleotide sequence, which is at least 70% and preferably 80%, especially at least 90% identical to a nucleotide sequence according to figure 7 (SEQ ID NO 5) and/or figure 10 (SEQ ID NO 5). Preferably these nucleotide sequences code for a splice variant of the p19 subunit of IL-23. Preferably these nucleotide sequences code for a regulator of IL-23.

Additionally, the invention comprises an amino acid sequence, which is at least 70% and preferably at least 80%, especially at least 90% identical to an amino acid sequence according to figure 9 (SEQ ID NO 6). Preferably these amino acid sequences represent the amino acid sequence of a splice variant of the p19 subunit of IL-23. Preferably these amino acid sequences form a regulator for IL-23 as described above.

Furthermore, the invention comprises an active agent especially for the treatment of diseases, wherein the active agent is the regulator of IL-23 as described above. Since the inventive regulator is able to influence bioactivity of IL-23, the active agent is especially useful for the treatment of diseases which are accompanied by reduced IL-23 activity, e.g. colitis, rheumatism and psoriasis.

In another embodiment of the invention an active agent for the treatment of diseases is provided, which influences the regulator of IL-23 as it is described above. In one embodiment said active agent increases the activity of the "regulator" of IL-23. Such an active agent is especially useful for the treatment of diseases which are accompanied by low IL-23 activities, e.g. in several types of cancer. In another embodiment the active agent reduces the activity of the regulator of IL-23. Such an active agent is especially useful for the treatment of diseases which are accompanied by an increased IL-23 activity like e.g. Psoriasis, Multiple Sclerosis, Rheumatoid Arthritis, Diabetes, Morbus Crohn, Ulcerative Colitis, Transplantation.

Preferably the active agent is a substance which interacts with the regulator thereby blocking its stimulating activity. In another embodiment the active agent is an inactive variant of the regulator, which competes for substrates with the functional regulator and/or influences the forming of functional protein complexes. Such functional protein complexes may be built of IL-23 and the regulator and/or other components.

Examples of diseases which could be effectively treated with an active agent, which increases the activity of the regulator of IL-23, are neurodegenerative diseases, cardiovascular diseases, immunological diseases, autoimmune diseases, inflammation diseases and/or cancer. By administering an active agent which reduces the activity of IL-23 as described above, the pathological condition is counteracted. In general all these (and other) diseases are preferred candidates for the treatment with an active agent according to the invention. One skilled in the art will known under which circumstances what kind of active agent has to be used. Consequently, it depends on the certain circumstances of the disease to be treated, if an active agent is chosen which increases the activity of the regulator of IL-23 or an active agent is chosen which reduces said activity.

In general, all regulators of IL-23 as also active agents influencing the activity of the regulators of IL-23 can be used to influence the (bio)activity of IL-23.

According to the invention activators of the novel splice variant of the p19 subunit of IL-23 can be used e.g. as therapeutical agents (for instance as therapeutical proteins) to treat e.g. Psoriasis, Multiple Sclerosis, Rheumatoid Arthritis, Diabetes, Morbus Crohn, Ulcerative Colitis, Transplantation. In addition to the already described activators of the inventive matter, also e.g. genetically modified (novel splice variant) of the p19 subunit of IL-23 and/or the p19 subunit by its own (the nucleotide and/or amino acid sequences) can be used as deactivator of IL-23. Such activators may be e.g. antisense oligos or antibodies against the splice variant of the p19 subunit of IL-23.

On the other side inhibitors of the novel splice variant of the p19 subunit of IL-23 can be used. e.g. as therapeutical agents (for instance as therapeutical proteins) to treat e.g. all types of cancer. Such inhibitors of the inventive matter would do the opposite of the antisense oligos or antibodies against IL-23 or generally the blockade of the alternative splicing, i.e. the inhibition of formation of bioactive IL-23 should be canceled. Such inhibitors of the inventive matter of the p19 subunit of IL-23 can be used to treat the above mentioned diseases.

These active agents can be used according to one preferred embodiment to treat diseases or to manufacture a medicament or a pharmaceutical composition for treating diseases.

Furthermore, the invention comprises the use of an active agent as described above for the treatment of diseases and a method for the treatment of diseases, wherein at least one inventive agent as described above is administered. Regarding further details of said use and said method reference is made to the description above.

Additionally, the invention comprises a pharmaceutical composition which comprises at least one active agent as described above in an effective amount and if necessary at least one pharmaceutical carrier. Depending from the circumstances of the disease to be treated the pharmaceutical composition is intended for topical or systemical administration, for example.

Furthermore, the invention comprises the use of a regulator of IL-23 for the diagnosis of diseases, wherein the frequency of said regulator in the organism is analysed. As outlined above, several disorders are accompanied by an increased or decreased activity of IL-23 in comparison with non-pathological conditions. In many cases the altered IL-23 activity is caused by a changed activity and/or frequency of the regulator of IL-23, which is based on a splice variant as described above. For example in an animal model for multiple sclerosis, rheumatoid arthritis or cancer. Therefore, the invention comprises the use of the regulator for diagnosis of the respective diseases, especially in the early diagnosis of the diseases. In a preferred embodiment of the inventive use at least one substance is used, which interacts with the regulator of IL-23. The regulator of IL-23 is preferably a regulator as described above.

In a preferred embodiment of the inventive use said interacting substance is an antibody which interacts with the regulator, preferably on the level of protein-protein or protein-peptide interaction. In general any antibody specific for the regulator is suitable in the inventive manner, for example polyclonal antibodies and/or monoclonal antibodies, as will be obvious for an expert in the art. In a preferred embodiment of this inventive use the specific antibody is placed on an array which permits a fast and easy performance of the diagnostic test, for example the antibody is placed on a dip stick as it is known in the field of immunodiagnosis.

In another embodiment of the inventive use the interacting substance is a nucleic acid molecule, which hybridises with at least a portion of a nucleic acid sequence coding for the regulator. This embodiment is especially preferred because such hybridisation probes are cheap and easy to obtain and the diagnostic test can be performed by common methods. For example the hybridisation probe, i.e. the nucleic acid molecule, which hybridises with at least a portion of the nucleic acid sequence coding for the regulator, is placed on a DNA chip, and the complete diagnostic reaction is preferably performed in an automated manner. In this embodiment the inventive use is especially useful for fast diagnosis of different diseases.

Preferred examples of said diseases are connected with a disturbance of IL-23 activity, for example said diseases are neurodegenerative diseases like amyotrophic lateral sclerosis, cardiovascular diseases like heart failure, immunological diseases, autoimmune diseases, inflammation diseases and/or cancer. All these diseases are examples for diseases connected with a disturbance of IL-23 activity like Psoriasis, Multiple Sclerosis, Rheumatoid Arthritis, Diabetes, Morbus Crohn, Ulcerative Colitis, Transplantation etc..

Furthermore, the invention comprises a kit, which comprises at least one substance which is able to interact with the regulator of IL-23. This kit is especially useful for diagnosis as described above. In a preferred embodiment of said kit the kit comprises at least one specific antibody for the regulator. In another embodiment of the invention the kit comprises at least one nucleic acid molecule which is able to hybridise with at least a portion of a nucleic acid sequence coding for the regulator.

Furthermore the invention comprises a method for searching substances which influence the activity of IL-23. This method is characterized in that a splice variant of the p19 subunit of IL-23 is used to identify and/or isolate substances which are able to interact with the splice variant itself and/or with IL-23. In a preferred embodiment of this method the activity of IL-23 is measured in vitro, whereby the influence of the regulator is analysed with or without (as control) additional substances, which possibly influence the activity of IL-23 via the regulator. In another preferred embodiment of this method the complex formation between at least IL-23, the regulator and the substance possibly influencing the activity of IL-23 is used as read-out system. Regarding further details of the regulator used in this method reference is made to the description above.

Finally the invention comprises an active agent for the treatment of diseases, which is identified and/or isolated by a method as described above.

The described features and further features of the invention read in greater detail from the following description of the figures in conjunction with the examples and the subclaims, whereby each of the individual features could be realized alone or in combination with each other.

In the figures it is shown:

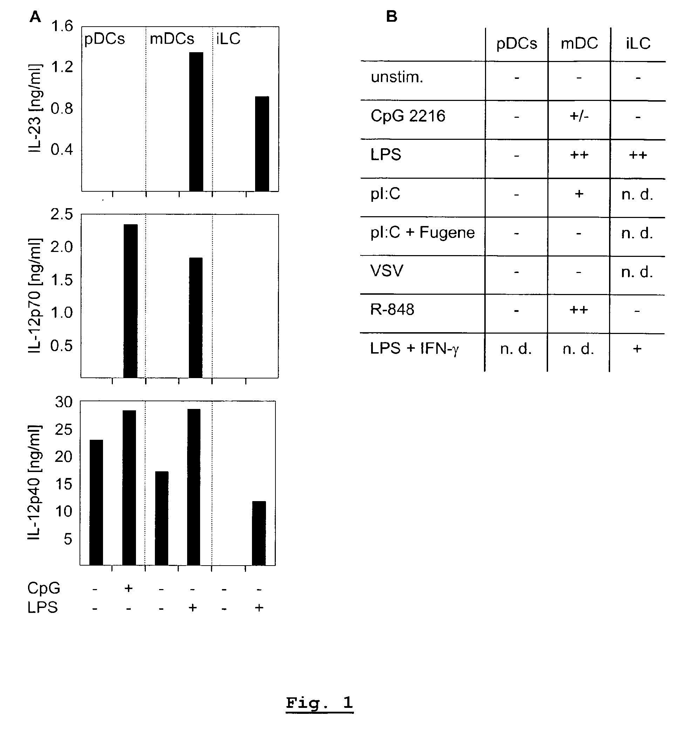

- Fig. 1: **Only dendritic cells of the myeloid lineage secrete IL-23 heterodimers.** While pDCs did not secrete IL-23, both myeloid DC types produced considerable amounts of this cytokine (**A**). *In vitro* differentiated BM-pDCs were treated with CpG 2216 (10 µg/ml) whereas BM-mDCs and iLC cells were stimulated with LPS (10 µg/ml). Untreated cells served as control. 24 h after stimulation cell-free supernatant was collected, was concentrated 7-fold and analysed by ELISA methods for IL-23, IL-12p70, and IL-12 p40 (**B**). BM-pDCs, BM-mDCs, and iLC were treated with the indicated stimuli or were left untreated (unstim.). 24 h after stimulation cell-free supernatant was analysed for IL-23 accumulation with an ELISA method. As a control 10 ng recombinant murine p40 protein was assayed to confirm specificity of the ELISA method for IL-23 heterodimer (not shown). n.d.: not done; -: <20 pg/ml; +/-: 20-30 pg/ml; +: 30-40 pg/ml; ++: >40 pg/ml. All data shown are representative for at least three independent experiments in duplicates.
- Fig. 2: **Detailed analysis of cytosolic and released IL-23 protein subunit expression by different types of DC.** *In vitro* differentiated BM-pDCs and BM-mDCs and iLC cells were stimulated as described in Fig. 1 or were left untreated under serum-free conditions. After 24 hours of stimulation supernatants (SN) were collected and concentrated and subsequently cytosolic (C) protein isolated from the cells for Western blot analysis with p40 or p19 specific antibodies (see Methods for details).

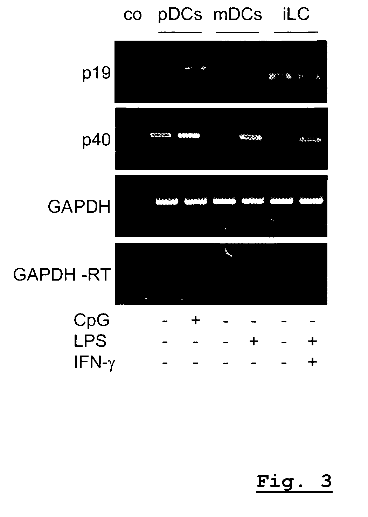

- Fig. 3: **mRNA regulation of IL-23 subunits in mDCs and pDCs.** *In vitro* differentiated BM-pDCs were stimulated with CpG 2216 (10 µg/ml) or were left untreated whereas *in vitro* differentiated BM-mDCs were stimulated with LPS (10 µg/ml); controls were left untreated. iLC were stimulated with LPS (1 µg/ml) and IFN-γ (10 ng/ml) or were left untreated as control. After 24 hours of stimulation cells were harvested and total RNA was isolated and reverse transcribed. The mRNA content of all samples was normalized by GAPDH-specific RT-PCR. As control, confirming the absence of genomic DNA, PCRs were performed using RNA before reverse transcription as a template (GAPDH-RT). To investigate IL-23 subunits expressed, primer pairs amplifying p19 or p40 were used ('co'= water controls for PCR). Results are representative for three to seven experiments.

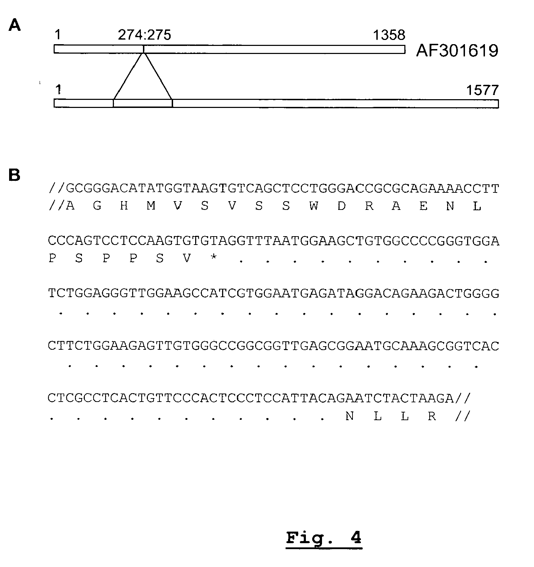

- Fig. 4: **Identification of a novel p19 splice variant.** Both bands detected by RT-PCR using p19 specific primers (see Fig. 3) were cloned and subsequently sequenced. Compared to the gene bank entry AF301619 (bp 1 to bp 1358), the longer PCR product showed an insertion of an alternatively spliced exon of 219 bp between bp 274 and bp 275 of the known mRNA sequence. Given in (**A**) is an overview of the structure of the alternative p19 mRNAs (numbers indicate bp). Shown in (**B**) is the p19 sequence from bp 263 to 286. The sequence of the alternative exon is highlighted by gray. The resulting protein sequence is indicated; * marks the stop within the novel protein sequence.

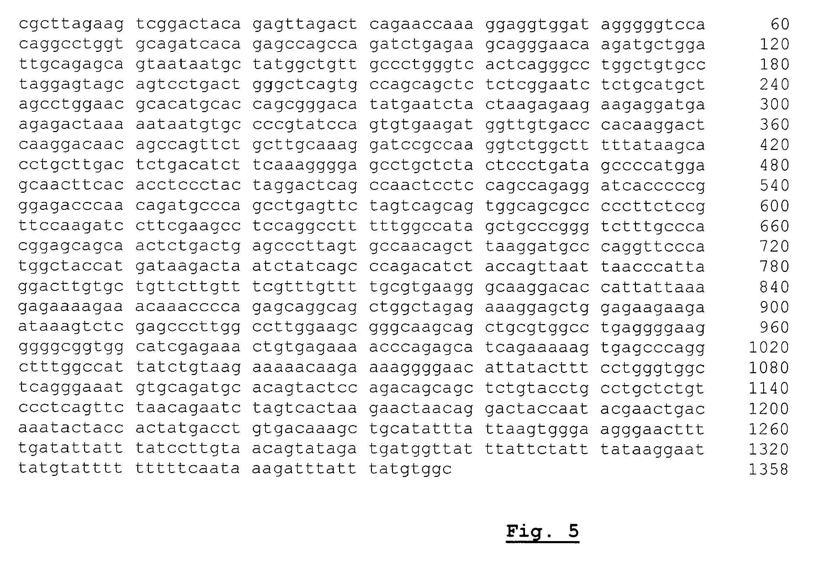

- Fig. 5: Nucleotide sequence of Mus musculus (Mouse) Interleukin-23 p19 subunit (cds 113-703).

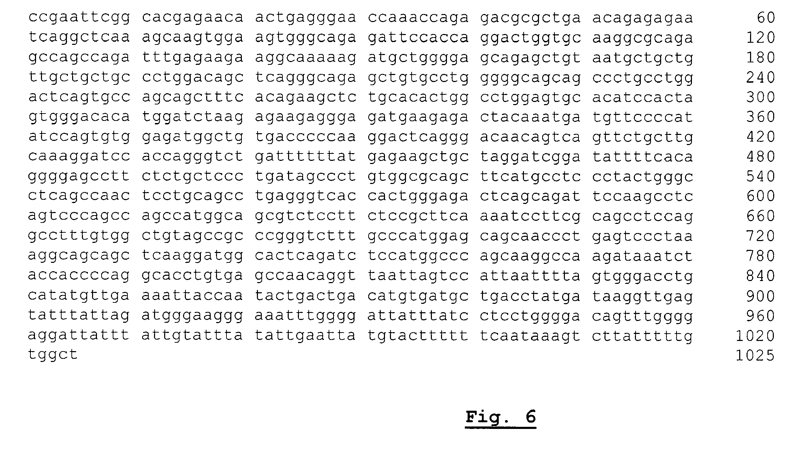

- Fig. 6: Nucleotide sequence of Homo sapiens Interleukin-23 p19 subunit (cds 151-720).
- Fig. 7: Nucleotide sequence of the inventive splice variant of the Interleukin-23 p19 subunit (cds 113-331, including an additional exon with a stop codon for protein translation).

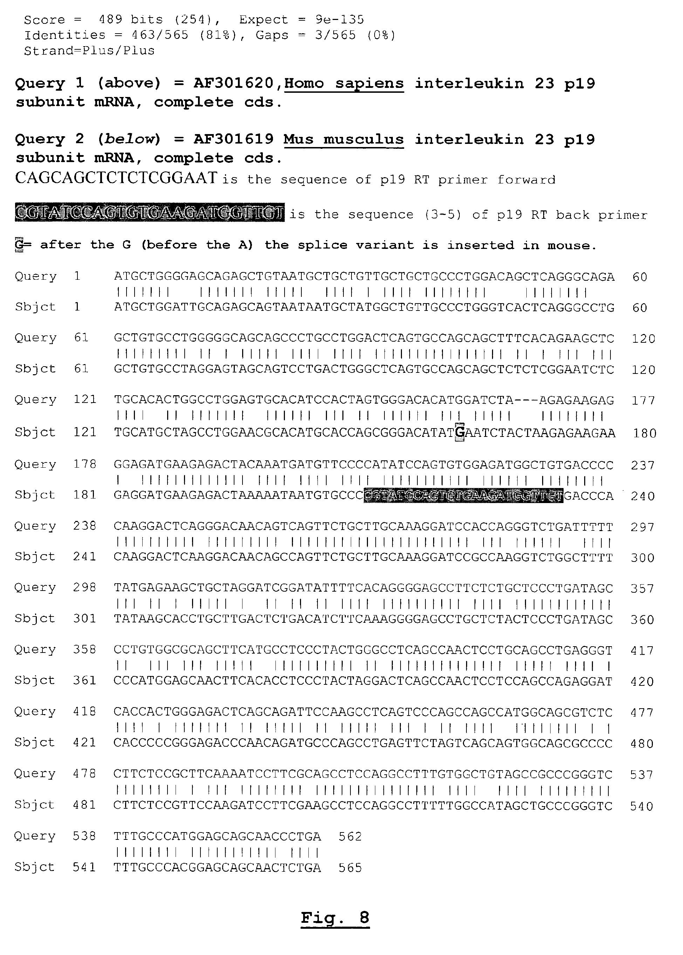

- Fig. 8: Nucleotide sequence comparison between the Interleukin-23 p19 subunits of Mus musculus and Homo sapiens (started at the start-codon ATG), analyzed by the BLASTN 2.2.14 software. The figure shows that there is a 81% identity between the known p19 subunits of interleukin-23 in these different species.
- Fig. 9: Protein sequence of Mus musculus (Mouse) Interleukin-23 p19 subunit and the inventive splice variant of said subunit. The figure shows that between M (methionine) and N (asparagine) of the p19 subunit the splice variant is located with the additional amino acid sequence.
- Fig. 10: Nucleotide sequence of the inventive splice variant of the Interleukin-23 p19 subunit "embedded" in the "normal" nucleotide sequence of p19. The figure shows the additional nucleotide sequence of the splice variant and the TAG-stopcodon in the splice variant.

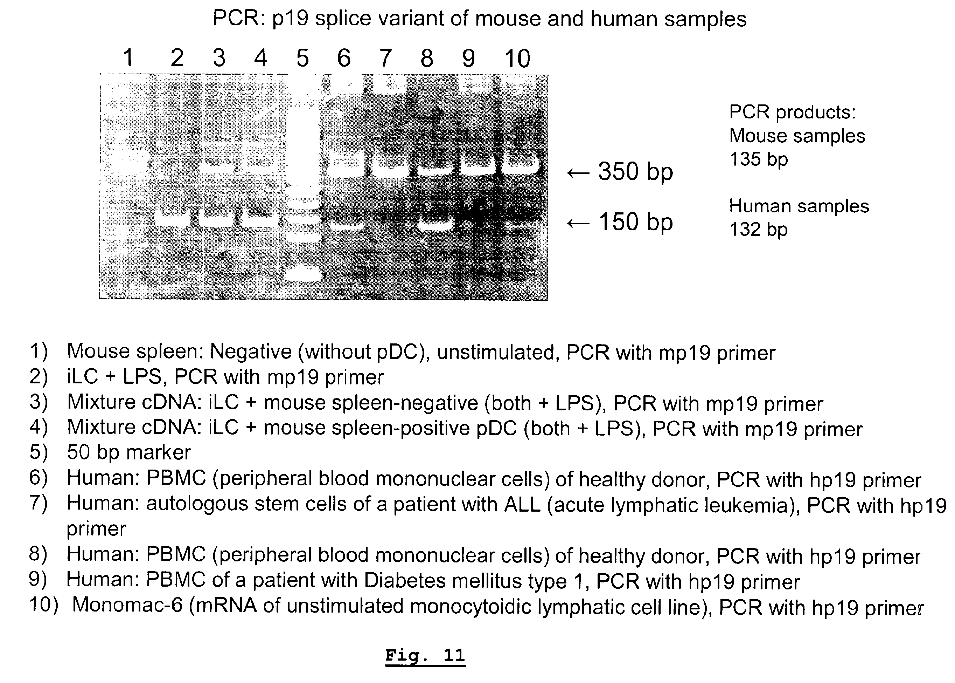

- Fig. 11: **mRNA regulation of IL-23 subunits in mouse spleen dendritic cells and in human cells.** Isolated mouse spleen dendritic cell fractions were left untreated or stimulated with 1 µg/ml LPS. After 20 hours, total RNA was isolated and reverse transcribed. To investigate p19 expression, primer pairs amplifying p19 were used. For identification of p19 in human cells, RNA of unstimulated PBMC of different donors, of autologous stem cells of an ALL-patient, or RNA from Monomac-6 cells was isolated and reverse transcribed. To investigate p19 expression, primer pairs amplifying human p19 were used.

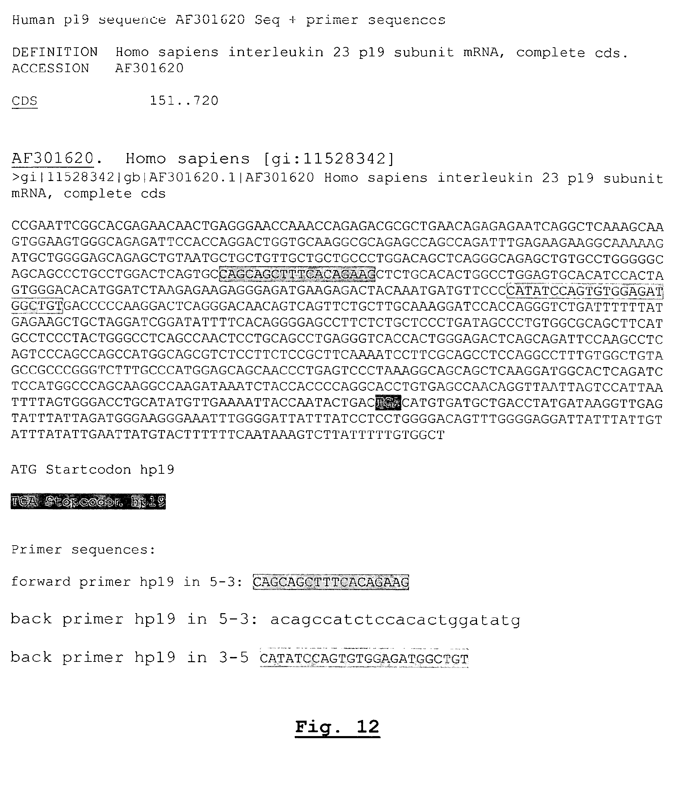

- Fig. 12: Nucleotide sequence of the human Interleukin-23 p19 subunit. The figure shows the ATG startcodon, the TAG-stopcodon as also the position of the used primers.

### Materials and Methods

### Mice and viruses

All mice were kept under specific pathogen free (SPF) conditions at the Zentrale Tierhaltung of the Paul-Ehrlich-Institute. C57BL/6 mice were purchased from Charles River. Mouse experimental work was carried out using 8- to 12-week old female mice. Vesicular stomatitis virus (VSV) Indiana, Mudd-Summers isolate, was originally obtained form D. Kolakofsky, University of Geneva, Geneva, Switzerland. VSV was grown on BHK-21 cells and plaqued on Vero cells.

### Cell culture

Mouse bone marrow (BM) derived pDCs and mDCs were generated as previously described (Bjorck, 2001). In brief, BM cells were isolated by flushing femur and tibia with RPMI supplemented with 10% FCS. Upon red blood cell lysis, cells were washed and seeded in medium supplemented with GM-CSF (100 ng/ml; R&D Systems) or Flt3-L (100 ng/ml; R&D systems) to generate mDCs and pDCs, respectively. The preparation, characterization and maintenance of the immature LC cell line XS52 (iLC) has been described before (Xu et al., 1995; Radeke et al., 2005). Plasmacytoid dendritic cells were isolated from mouse spleen with Anti-mPDCA-1 MicroBeads (Miltenyi Biotec, Bergisch Gladbach) according to the manufacturer's instructions. The pDC fraction binding to the Anti-mPDCA-1 MicroBeads was termed as the "positive fraction", while the remaining fraction without pDCs was termed "negative fraction". Both fractions were kept separately in iLC-medium supplemented with FCS for 24 hours, while stimulation experiments were performed in FCS-free medium. For experiments with human cells, PBMC of different donors, autologous stem cells from an ALL-patient, or Monomac-6 cells were used.

### Stimulation and cytokine analysis

For stimulation experiments, 1x10⁶ mDCs, iLC or pDCs were seeded in 24-well culture plates in 1 ml medium. The following stimuli were used: CpG 2216 (10 µg/ml; synthesized by Sigma ARK genosys (Steinheim, Germany), R-848 (5 µM; 3M Pharmaceuticals), LPS and poly-I:C (both 10 µg/ml; Sigma-Aldrich, Germany), and VSV at multiplicities of infection (MOIs) 10. For transfection of 2 µg polyI:C the reagent Fugene (Roche, Basel, Switzerland) was used according to manufacturer's instructions. For stimulation of iLC 1 µg/ml LPS and 10 ng/ml murine IFN-γ (Strathmann, Hamburg, Germany) was applied after switching to serum-free conditions. After 24 hours of stimulation cell-free supernatants were collected and analyzed for IL-23p19/p40, IL-12p70, or IL-12p40 by commercial or internally developed ELISA methods (eBioscience, San Diego, USA). Both for ELISA and Western blots (see below) supernatants were concentrated seven-fold using Centriprep YM10 (Millipore, Schwalbach, Germany) 10kDa filter spin concentrators.

### RT-PCR

Total RNA was prepared using TRIZOL reagent (Invitrogen, Carlsbad, CA) according to manufacturer's instructions. RNA was incubated with DNase (Roche) for 15 min at 37°C and cDNA was prepared by using SuperScript II (Invitrogen) according to manufacturer's instructions. PCRs were performed using the following primers (sequences listed in 5' to 3' orientation):
GAPDH: accacagtccatgccatcac and tccaccaccctgttgctgta;
murine p19: CAGCAGCTCTCTCGGAAT and ACAACCATCTTCACACTGGATACG;
murine p40: TCTTTGTTCGAATCCAGCG and GAAAACTGGAAAAAGCCAACC.

Primer sequences for the human p19 (spanning the inserted exon) were:
Human p19 forward: CAGCAGCTTTCACAGAAG
Human p19 back: ACAGCCATCTCCACACTGGATATG

The mRNA content was normalized by RT-PCR analysis with GAPDH-specific primers. PCRs using RNA before reverse transcription (-RT) as template confirmed the absence of genomic DNA from the samples.

### Western Blotting

As described above supernatants were harvested after 24 hrs under serum-free conditions and concentrated seven-fold. Cytosolic fractions were prepared essentially as described before (Radeke et al., 1994; Radeke et al., 2005). Briefly, following treatment semi-adherent DC were gently scraped on ice, samples spun down and supernatants collected, concentrated 7-fold by Centriprep filters 10 kDa and stored at -80°C. Following 1x wash with PBS at 4°C lysis buffer, PBS, 1mM HEPES, pH 7.4, containing proteinase inhibitor cocktail (bacitracin 0.7mM, and leupeptin; iodoacetamide, benzamidine, PMSF, 1.0mM, respectively, all from Sigma-Aldrich) was added and cellular pellets sonicated for 3x10sec (at 50 Watt) on ice. After spinning down the nuclei supernatants were subjected to ultracentrifugation at 100,000xg, 30min at 4°C and supernatant cytosolic fractions carefully removed and stored at -80°C until further processing. Protein concentration was determined using a standard BCA protein assay system (Pierce, Rockford, IL) and equal amounts of protein, usually 4µg per lane, subjected to 12.5% polyacrylamide SDS-PAGE under reducing conditions. Detection of p40 and p19 proteins was performed using murine p19- or p40-specific antibodies (R&D Systems, Wiesbaden, Germany). Secondary anti-goat-HRP antibodies (Dako, Hamburg, Germany) and streptavidin-HRP (R+D Systems) were applied. For visualization a luminescence system with the ECL reagent (Amersham Biosciences, Little Chalfont, UK) was used as described before (Radeke et al., 2005).

### Statistics

All experiments, ELISA in duplicates, Western blots, and RT-PCRs were performed at least three, and up to seven times and representative experiments are shown.

### Results

### Myeloid but not plasmacytoid DCs secrete IL-23

Dendritic cells (DCs) have been described as one major source of a series of pro-inflammatory and anti-inflammatory cytokines. In particular, plasmacytoid DCs (pDCs) produce enormous amounts of type I interferon upon bacterial or viral infection while conventional DCs seem to mount strong TNF-α response upon LPS stimulation. To test which DC lineage, plasmacytoid or myeloid DCs (mDCs), produce IL-23 upon stimulation we cultivated bone marrow (BM) cells in the presence of GM-CSF or Flt3-L to differentiate BM-mDCs or BM-pDCs, respectively. After 8 days of cultivation in the presence of Flt3-L, more than 80% of the bone marrow cultures were CD11c+B220+ and thus shared characteristics with pDC that are also referred to as IFN-α producing cells. Using GM-CSF as a culture additive, after 8 days about 75% of bone marrow cells were CD11b+B220- and thus showed characteristics of myeloid DCs (data not shown). BM-pDCs, BM-mDCs, or a cell line of immature Langerhans cells (iLC) were stimulated with CpG containing oligodeoxynucleotide (ODN) 2216 or LPS, respectively. While as expected IL-12p70 was produced by pDCs and mDCs but not by iLC, in contrast, IL-23 was only secreted by cells of the myeloid lineage upon stimulation (Fig. 1A). As measured by a specific ELISA the p40 subunit of IL-12/-23 was secreted by all cell types tested at least under stimulating conditions (Fig. 1A, lower panel). Subsequently, in order to rule out the possibility that the failure of pDC to secrete IL-23 was due to inadequate TLR stimulation DC were stimulated with ligands of the Toll-like receptors (TLR) 3, 4, 7 and 9 i.e poly I:C, LPS, R-848 or CpG ODN systematically and 24 h later cell-free supernatants were analyzed for IL-23 heterodimer accumulation by an ELISA method. These experiments indicated that under no conditions tested pDCs secreted IL-23, whereas mDCs were able to produce IL-23 when treated with LPS, poly I:C or R-848 (Fig. 1B). Supporting the concept of a lineage-specific effect stimulation of iLC with TLR 7/8 ligand R-848 failed to induce a significant IL-23 secretion. As a further control for our assays system and in line with recently published data we confirmed that IFN-γ reduced LPS augmented IL-23 secretion in iLC (Fig. 1B).

### pDCs are unable to produce the p19 protein subunit

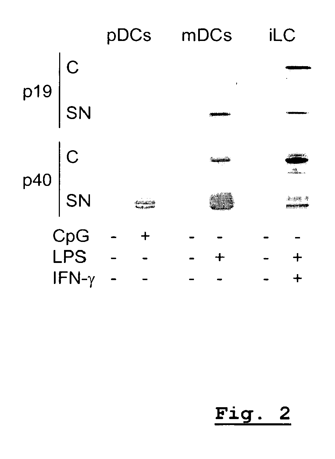

To investigate why myeloid cells but not pDCs were able to secrete the heterodimeric p19/p40 bioactive IL-23 protein, Western blot analyses of both cytosol and supernatants for the two IL-23 subunits p19 and p40 were performed. Cells were kept under serum free conditions, and were treated with their optimal stimuli that are CpG in the case of pDCs, LPS in the case of mDCs, and compared to untreated cells. 24h after stimulation cell-free supernatants and cytosolic fractions were harvested and used in Western blot analyses. As shown in Figure 2, neither in the supernatant nor in the cytosolic fraction of bone marrow-derived pDCs the p19 protein could be detected. In contrast, BM-mDCs and iLC stimulated with LPS or LPS plus IFN-γ, respectively, were able to produce and release the monomeric p19 subunit. Compared to iLC, BM-mDCs were even more efficient in secreting p19, and no cytosolic protein could be detected within these cells, probably reflecting the higher IL-23 heterodimer levels in the supernatants of these cells measured by ELISA (see Fig. 1). The IL-12/-23 subunit p40 was detected upon stimulation of plasmacytoid and myeloid cells, whereas the protein was absent in supernatants or the cytosol of unstimulated cells.

### IL-23 p19 and p40 mRNA expression differs between plasmacytoid and myeloid DC

To gain more insight into transcription and translation of heterodimeric IL-23 in DC subsets RT-PCR analyses were performed. pDCs, mDCs and iLC cells were treated with CpG, LPS or LPS plus IFN-γ, respectively, and controls were left untreated. Upon 24 h of stimulation, cells were harvested and total RNA was isolated. Samples were normalized to a GAPDH-specific RT-PCR and GAPDH-specific RT-PCR with RNA as template confirmed the absence of genomic DNA. The p19 specific RT-PCR indicated that p19 mRNA was expressed constitutively in all cell types tested, irrespective of whether the cells were activated or not (Fig. 3). This new finding suggests that the IL-23 subunit p19 has to be regulated on a posttranscriptional level. Remarkably pDCs that are unable to form detectable amounts of the p19 protein, also expressed p19 mRNA constitutively. In contrast, p40 mRNA was regulated differentially in plasmacytoid and myeloid dendritic cells. pDCs constitutively expressed p40 mRNA (Fig. 3) but translated the mRNA only upon stimulation (Fig. 2). In contrast, BM-mDCs and iLC cells upregulated p40 mRNA upon stimulation (Fig. 3). Thus, expression of the IL-23 subunits p19 and p40 is regulated quite different both with respect to the cell type and the subunit.

### Detection of a novel p19 splice variant

Using p19 specific primers, RT-PCRs revealed two amplification products in pDCs and mDCs. However, the longer PCR product was not detectable in iLC cells (Fig. 3). To address the length heterogeneity both bands were cloned and sequenced. DNA sequence analysis revealed that both bands represented p19-derived sequences. Compared to the gene bank entry AF301619, the longer clones contained a splice variant, in which an additional exon has not been spliced out. This additional exon was 219 bp long and encoded for a stop codon after 18 novel amino acids leading to a shortened protein (Fig. 4). PCRs with RNA extracts devoid of reverse transcription confirmed the absence of genomic DNA contaminations from all samples.

While mouse bone marrow derived DCs contain both, and p19 splice variant RNAs (see Fig. 3), in contrast dendritic cells isolated from mouse spleen (pDCs as well as mDCs) only contain the p19 splice variant (Fig. 11). By PCR with a mixture of iLC and spleen dendritic cells, both p19 mRNA types were detected and confirmed the specificity of the p19 primers (Fig. 11). In addition, also RNAs of human cells from different origin show an additional band after PCR with p19-specific primers. A difference was found between healthy donors whose PBMC contain p19 and an additional p19 splice variant, while in PBMC of patients either with Diabetes mellitus type 1 or with ALL (acute lymphatic leukemia), only the p19 splice variant was detected. Therefore, this newly identified p19 splice product in human cells shows differences between healthy and sickly patients.

Plasmacytoid dendritic cells (pDCs) have been implicated in both, highly inflammatory reactions to viral invasion with a unique cytokine profile including type I interferons (IFNs) (Novelli and Casanova, 2004; Conzelmann, 2005), and as regulatory cells dampening myeloid dendritic cell (mDC) driven lung inflammation (de Heer et al., 2005; Lambrecht, 2005). While in most instances they show a rather limited capacity for antigen presentation they are effective activators of CD8 T cells following viral activation (McKenzie et al., 2006;Alber et al., 2006). Under physiological or acute inflammatory conditions they exhibit a migratory profile limited to the central lymphatic compartments. However, it has been convincingly shown that pDC extravasate to chronically inflamed sites possibly using a specific set of chemokine receptor and ligand (Vermi et al., 2005). So far, conflicting data have been presented concerning the effects of type I IFNs on the regulation of IL-12p70 (Byrnes et al., 2001). Given the decisive role for DC-derived IL-12 and IL-23 for the translation of injurious bacterial, viral or fungal attacks into a proper pro-inflammatory adaptive T cell-response (Langrish et al., 2004; McKenzie et al., 2006; Alber et al., 2006), especially with respect to the new independent role of the IL-23 / IL-17 axis (Wynn, 2005), this invention analyzed the relative contribution of mDC versus pDC to the T cell differentiating signals of bioactive IL-12 and IL-23.

So far it has not been examined whether murine pDC (BM-derived, Flt-3-ligand-expanded, CD11c+B220+) are able to produce IL-23. Here for the first time it is demonstrated that murine pDC, while being capable to express the IL-12/-23 p40 and p19 mRNA, did not proceed to translation or to assembly of a bioactive IL-23 heterodimer. Thus, it is shown that the regulation of hazardous IL-23 is governed not only at the transcriptional but also very effectively at the translational or post-translational level.

The identified and characterized splice variant of p19 is of high interest, especially with respect to the novel regulatory step at pre-translational (splice regulators), translational or post-translational level. A truncated p19 protein worked as a molecular block for an effective assembly of the IL-23 heterodimer.

Since IL-23 has been reported to cause differentiation of a unique subset from naive to highly pro-inflammatory IL-17 secreting T cells (Harrington et al., 2005; Park et al., 2005; Wynn, 2005), the failure of pDCs to generate active IL-23 reflects the reduced ability of this DC subset to induce the specific IL-17-producing T cells ("Th17"). In contrast, stimulation of pDCs with CpG-containing oligodeoxynucleotide (ODN) induced considerable amounts of IL-12p70 almost reaching the range of LPS activated mDC, while immature Langerhans cells under no condition tested assembled bioactive IL-12p70 but rather high amounts of bioactive heterodimeric IL-23.

Because pDC have often been associated with a modulatory, in some investigations even a tolerogenic role, during transition from innate to adaptive immune response (de Heer et al., 2005; de Heer et al., 2004), the presented data show that IL-12 has anti-inflammatory functions, and that other products like IL-10 and type 1 IFNs play a important role for this anti-inflammatory activity. However, it can not be ruled out that pDC release of IL-12p70 may take part in a regulatory loop. In line with the finding, that IL-12/IFN-γ are carrying an anti-inflammatory activity, it has been shown in models of multiple sclerosis and arthritis that removal of IL-12/IFNγ exacerbates autoimmune disease (Becher et al., 2002; Gran et al., 2002; Murphy et al., 2003).

Alternatively pDCs producing a truncated p19 based on the splice variant detected in this invention might directly block the assembly of bioactive IL-23 not only in pDC but also -as a transcellular mechanism- in the high IL-23 producing myeloid DC.

In this invention it is shown that mDCs produce IL-23 upon stimulation with many different TLR ligands including poly I:C (artificial dsRNA; ligand for TLR3, but also for cytoplasmic non-TLR sensors), CpG ODN 2216 (dsDNA; ligand for TLR9), LPS (component of gram-negative bacteria; ligand for TLR4), and TLR7/8 ligand R-848 (artificial ssRNA). The broad range of different stimuli able to induce IL-23 reflect the prominent role of DCs from the myeloid lineage for the production of bioactive IL-23. Interestingly, stimulation of mDCs with vesicular stomatitis virus (VSV), encoded by an ssRNA genome, did not induce IL-23 production. This is due to the viral escape mechanism activated by VSV in infected mDCs (Novelli and Casanova, 2004). This viral escape involves blockage of host RNA export out of the nucleus by binding of viral M protein to cellular export molecules Nup/Rae.

At this point it is important to mention that our *in vitro* studies have been performed with isolated DC subpopulations. In the multicellular in vivo situation the induction of IL-23 is prevented in the presence of IL-4 and IL-10 or lowered by IFNγ and only becomes significant after blocking these cytokines (Harrington et al., 2005; Wynn, 2005). It can not be excluded that these cytokines are active inducers of the alternative splicing and thereby their anti-inflammatory activity is partly explained by the IL-23 regulator regulating activity.

Analogously, unlike inflammatory type 1 human macrophages, which indeed produce bioactive IL-23 and promote immunity to bacteria, type 2 macrophages do not generate and release IL-23 in response to microbial stimuli and instead produce IL-10 (Verreck et al., 2006). Furthermore, Pirhonen et al. showed that human macrophages produced IL-23 upon stimulation with Sendai virus. Upon co-stimulation of these cells with Sendai virus and IL-4, IL-23 production was abolished (Pirhonen et al., 2002). In addition to pro-inflammatory cytokines pDCs are known to secrete immunoregulatory cytokines including IL-10 or TNF-α (Lambrecht, 2005). While the phenotypic characterization of type 1 versus type 2 macrophages does not entirely correlate to the properties of mDC versus pDC the functional activity described for both, IL-23-negative type 2 macrophages and pDC is overlapping with respect to anti-inflammatory immune modulation by IL-23 negative APC (Lambrecht, 2005; Verreck et al., 2006). Thus, these findings show that the inability of pDCs to produce IL-23 is due to secretion of regulatory modulators inhibiting IL-23 protein translation and assembly in these cells. On the other hand dysregulated expression of bioactive IL-23 subunit p19 leads to the development of systemic inflammatory diseases like eczematous skin disease (Wiekowsky 2001; Kopp 2003).

In summary, the condensed set of data reveal an important piece of information necessary to understand the so-called "pro-inflammatory" arm of an adaptive immune response, which formerly was solely carried by T helper-1 type reactions and now comprises both IL-12 and IL-23 dependent adaptive immunity. It can be concluded that a concert of different DC types contributes to the finely tuned and coordinated immune response both in physiology and chronic inflammation.

### List of cited references :

1. Alber,G., Al-Robaiy, S., Kleinschek,M., Knauer,J., Krumbholz,P., Richter,J., Schoeneberger,S., Schuetze,N., Schulz,S., Toepfer,K., Voigtlaender,R., Lehmann,J., Mueller,U., 2006. Induction of immunity and inflammation by interleukin-12 family members. Ernst. Schering. Res Found. Workshop 107-127.
2. Becher,B., Durell,B.G., Noelle,R.J., 2002. Experimental autoimmune encephalitis and inflammation in the absence of interleukin-12. J. Clin. Invest 110, 493-497.
3. Bjorck,P., 2001. Isolation and characterization of plasmacytoid dendritic cells from Flt3 ligand and granulocyte-macrophage colony-stimulating factor-treated mice. Blood 98, 3520-3526.
4. Byrnes,A.A., Ma,X., Cuomo,P., Park,K., Wahl,L., Wolf,S.F., Zhou,H., Trinchieri,G., Karp,C.L., 2001. Type I interferons and IL-12: convergence and cross-regulation among mediators of cellular immunity. Eur. J. Immunol. 31, 2026-2034.
5. Conzelmann,K.K., 2005. Transcriptional activation of alpha/beta interferon genes: interference by nonsegmented negative-strand RNA viruses. J. Virol. 79, 5241-5248.
6. Cua,D.J., Sherlock,J., Chen,Y., Murphy,C.A., Joyce,B., Seymour,B., Lucian,L., To,W., Kwan,S., Churakova,T., Zurawski,S., Wiekowski,M., Lira,S.A., Gorman,D., Kastelein,R.A., Sedgwick,J.D., 2003. Interleukin-23 rather than interleukin-12 is the critical cytokine for autoimmune inflammation of the brain. Nature 421, 744-748.
7. de Heer,H.J., Hammad,H., Kool,M., Lambrecht,B.N., 2005. Dendritic cell subsets and immune regulation in the lung. Semin. Immunol. 17, 295-303.
8. de Heer,H.J., Hammad, H., Soullie,T., Hijdra, D., Vos,N., Willart,M.A., Hoogsteden,H.C., Lambrecht,B.N., 2004. Essential role of lung plasmacytoid dendritic cells in preventing asthmatic reactions to harmless inhaled antigen. J. Exp. Med. 200, 89-98.
9. Elkins,K.L., Cooper,A., Colombini,S.M., Cowley,S.C., Kieffer,T.L., 2002. In vivo clearance of an intracellular bacterium, Francisella tularensis LVS, is dependent on the p40 subunit of interleukin-12 (IL-12) but not on IL-12 p70. Infect. Immun. 70, 1936-1948.
10. Ghilardi,N., Kljavin,N., Chen,Q., Lucas,S., Gurney,A.L., de Sauvage,F.J., 2004. Compromised humoral and delayed-type hypersensitivity responses in IL-23-deficient mice. J. Immunol. 172, 2827-2833.
11. Gran,B., Zhang,G.X., Yu,S., Li,J., Chen,X.H., Ventura,E.S., Kamoun,M., Rostami,A., 2002. IL-12p35-Deficient Mice Are Susceptible to Experimental Autoimmune Encephalomyelitis: Evidence for Redundancy in the IL-12 System in the Induction of Central Nervous System Autoimmune Demyelination. J. Immunol. 169, 7104-7110.
12. Happel,K.I., Zheng,M., Young,E., Quinton,L.J., Lockhart,E., Ramsay,A.J., Shellito,J.E., Schurr,J.R., Bagby,G.J., Nelson,S., Kolls,J.K., 2003. Cutting edge: roles of Toll-like receptor 4 and IL-23 in IL-17 expression in response to Klebsiella pneumoniae infection. J. Immunol. 170, 4432-4436.
13. Harrington,L.E., Hatton,R.D., Mangan,P.R., Turner,H., Murphy,T.L., Murphy,K.M., Weaver,C.T., 2005. Interleukin 17-producing CD4+ effector T cells develop via a lineage distinct from the T helper type 1 and 2 lineages. Nat. Immunol. 6, 1123-1132.
14. Hunter,C.A., 2005. New IL-12-family members: IL-23 and IL-27, cytokines with divergent functions. Nat. Rev. Immunol. 5, 521-531.
15. Jefford,M., Schnurr,M., Toy,T., Masterman,K.A., Shin,A., Beecroft,T., Tai,T.Y., Shortman,K., Shackleton,M., Davis,I.D., Parente, P. , Luft,T., Chen, W., Cebon, J., Maraskovsky, E., 2003. Functional comparison of DCs generated in vivo with Flt3 ligand or in vitro from blood monocytes: differential regulation of function by specific classes of physiologic stimuli. Blood 102, 1753-1763.
16. Kleinschek,M.A., Muller,U., Brodie,S.J., Stenzel,W., Kohler,G., Blumenschein,W.M., Straubinger,R.K., McClanahan,T., Kastelein,R.A., Alber,G., 2006. IL-23 enhances the inflammatory cell response in Cryptococcus neoformans infection and induces a cytokine pattern distinct from IL-12. J Immunol 176, 1098-1106.
17. Kopp,T., Lenz,P., Bello-Fernandez,C., Kastelein,R.A., Kupper,T.S., Stingl,G., 2003. IL-23 production by cosecretion of endogenous p19 and transgenic p40 in keratin 14/p40 transgenic mice: evidence for enhanced cutaneous immunity. J. Immunol. 170, 5438-5444.
18. Krajina,T., Leithauser,F., Moller,P., Trobonjaca,Z., Reimann,J., 2003. Colonic lamina propria dendritic cells in mice with CD4+ T cell-induced colitis. Eur. J. Immunol. 33, 1073-1083.
19. Lambrecht,B.N., 2005. Dendritic cells and the regulation of the allergic immune response. Allergy 60, 271-282.
20. Langrish,C.L., McKenzie,B.S., Wilson, N.J., De Waal,M.R., Kastelein,R.A., Cua,D.J., 2004. IL-12 and IL-23: master regulators of innate and adaptive immunity. Immunol. Rev. 202, 96-105.
21. Leonard,J.P., Waldburger,K.E., Goldman,S.J., 1995. Prevention of experimental autoimmune encephalomyelitis by antibodies against interleukin 12. J. Exp. Med. 181, 381-386.
22. Lieberman,L.A., Cardillo,F., Owyang,A.M., Rennick,D.M., Cua,D.J., Kastelein,R.A., Hunter,C.A., 2004. IL-23 provides a limited mechanism of resistance to acute toxoplasmosis in the absence of IL-12. J. Immunol. 173, 1887-1893.
23. Liu,Y.J., 2005. IPC: professional type 1 interferon-producing cells and plasmacytoid dendritic cell precursors. Annu. Rev. Immunol. 23, 275-306.
24. Malfait,A.M., Butler,D.M., Presky,D.H., Maini,R.N., Brennan,F.M., Feldmann,M., 1998. Blockade of IL-12 during the induction of collagen-induced arthritis (CIA) markedly attenuates the severity of the arthritis. Clin. Exp. Immunol. 111, 377-383.
25. McIntyre,K.W., Shuster,D.J., Gillooly,K.M., Warrier,R.R., Connaughton,S.E., Hall,L.B., Arp,L.H., Gately,M.K., Magram,J., 1996. Reduced incidence and severity of collagen-induced arthritis in interleukin-12-deficient mice. Eur. J. Immunol. 26, 2933-2938.
26. McKenzie,B.S., Kastelein,R.A., Cua,D.J., 2006. Understanding the IL-23-IL-17 immune pathway. Trends Immunol. 27, 17-23.
27. Middleton,M.K., Rubinstein,T., Pure,E., 2006. Cellular and molecular mechanisms of the selective regulation of IL-12 production by 12/15-lipoxygenase. J. Immunol. 176, 265-274.
28. Murphy,C.A., Langrish,C.L., Chen,Y., Blumenschein,W., McClanahan, T., Kastelein,R.A., Sedgwick,J.D., Cua,D.J., 2003. Divergent pro- and antiinflammatory roles for IL-23 and IL-12 in joint autoimmune inflammation. J. Exp. Med. 198, 1951-1957.
29. Novelli,F., Casanova,J.L., 2004. The role of IL-12, IL-23 and IFN-gamma in immunity to viruses. Cytokine Growth Factor Rev. 15, 367-377.
30. Oppmann,B., Lesley,R., Blom,B., Timans,J.C., Xu,Y., Hunte,B., Vega,F., Yu,N., Wang,J., Singh,K., Zonin,F., Vaisberg,E., Churakova,T., Liu,M., Gorman, D., Wagner,J., Zurawski,S., Liu,Y., Abrams,J.S., Moore,K.W., Rennick,D., Waal-Malefyt,R., Hannum,C., Bazan,J.F., Kastelein,R.A., 2000. Novel p19 protein engages IL-12p40 to form a cytokine, IL-23, with biological activities similar as well as distinct from IL-12. Immunity. 13, 715-725.
31. Parham, C., Chirica, M. , Timans, J., Vaisberg, E., Travis, M., Cheung,J., Pflanz,S., Zhang,R., Singh,K.P., Vega,F., To,W., Wagner,J., O'Farrell, A.M., McClanahan,T., Zurawski,S., Hannum,C., Gorman,D., Rennick,D.M., Kastelein,R.A., de Waal,M.R., Moore,K.W., 2002. A receptor for the heterodimeric cytokine IL-23 is composed of IL-12Rbeta1 and a novel cytokine receptor subunit, IL-23R. J. Immunol. 168, 5699-5708.
32. Park,H., Li,Z., Yang,X.O., Chang,S.H., Nurieva,R., Wang,Y.H., Wang,Y., Hood,L., Zhu,Z., Tian,Q., Dong,C., 2005. A distinct lineage of CD4 T cells regulates tissue inflammation by producing interleukin 17. Nat. Immunol. 6, 1133-1141.
33. Pirhonen,J., Matikainen,S., Julkunen, I., 2002. Regulation of virus-induced IL-12 and IL-23 expression in human macrophages. J. Immunol. 169, 5673-5678.
34. Radeke,H.H., Gessner,J.E., Uciechowski,P., Magert,H.J., Schmidt,R.E., Resch,K., 1994. Intrinsic human glomerular mesangial cells can express receptors for IgG complexes (hFc gamma RIII-A) and the associated Fc epsilon RI gamma-chain. J. Immunol. 153, 1281-1292.
35. Radeke,H.H., von, W. H. , Stoidtner,K., Sauer,B., Hammer,S., Kleuser,B., 2005. Overlapping signaling pathways of sphingosine 1-phosphate and TGF-beta in the murine Langerhans cell line XS52. J. Immunol. 174, 2778-2786.
36. Sheibanie,A.F., Tadmori, I., Jing,H., Vassiliou,E., Ganea,D., 2004. Prostaglandin E2 induces IL-23 production in bone marrow-derived dendritic cells. FASEB J. 18, 1318-1320.
37. Smits,H.H., Van Beelen,A.J., Hessle,C., Westland,R., De Jong,E., Soeteman,E., Wold,A., Wierenga,E.A., Kapsenberg,M.L., 2004. Commensal Gram-negative bacteria prime human dendritic cells for enhanced IL-23 and IL-27 expression and enhanced Th1 development. Eur. J. Immunol. 34, 1371-1380.
38. Vermi,W., Riboldi,E., Wittamer, V., Gentili,F., Luini,W., Marrelli,S., Vecchi,A., Franssen,J.D., Communi,D., Massardi,L., Sironi, M., Mantovani, A., Parmentier, M., Facchetti, F., Sozzani,S., 2005. Role of ChemR23 in directing the migration of myeloid and plasmacytoid dendritic cells to lymphoid organs and inflamed skin. J. Exp. Med. 201, 509-515.
39. Verreck,F.A., de,B.T., Langenberg,D.M., van der,Z.L., Ottenhoff,T.H., 2006. Phenotypic and functional profiling of human proinflammatory type-1 and anti-inflammatory type-2 macrophages in response to microbial antigens and IFN-{gamma}- and CD40L-mediated costimulation. J. Leukoc. Biol. 79, 285-293.
40. Wiekowski,M.T., Leach,M.W., Evans,E.W., Sullivan,L., Chen,S.C., Vassileva,G., Bazan,J.F., Gorman,D.M., Kastelein,R.A., Narula,S., Lira,S.A., 2001. Ubiquitous transgenic expression of the IL-23 subunit p19 induces multiorgan inflammation, runting, infertility, and premature death. J. Immunol. 166, 7563-7570.
41. Wynn,T.A., 2005. T(H)-17: a giant step from T(H)1 and T(H)2. Nat. Immunol. 6, 1069-1070.
42. Xu,S., Bergstresser,P.R., Takashima, A., 1995. Phenotypic and functional heterogeneity among murine epidermal-derived dendritic cell clones. J. Invest Dermatol. 105, 831-836.
43. Ye,P., Rodriguez,F.H., Kanaly,S., Stocking,K.L., Schurr,J., Schwarzenberger,P., Oliver,P., Huang,W., Zhang,P., Zhang,J., Shellito,J.E., Bagby,G.J., Nelson,S., Charrier,K., Peschon,J.J., Kolls,J.K., 2001. Requirement of interleukin 17 receptor signaling for lung CXC chemokine and granulocyte colony-stimulating factor expression, neutrophil recruitment, and host defense. J. Exp. Med. 194, 519-527.

## Claims

1. Regulator of IL-23 (interleukin-23), **characterized in that** it is based on a splice variant of the p19 subunit of IL-23.

2. Regulator of IL-23 according to claim 1, **characterized by** an amino acid sequence at least 70% identical to an amino acid sequence according to figure 9 (SEQ ID NO 6).

3. Regulator of IL-23 according to claim 1 or 2, **characterized in that** it is coded by a nucleotide sequence at least 70% identical to a nucleotide sequence according to figure 7 (SEQ ID NO 5) and/or to figure 10 (SEQ ID NO 5).

4. Nucleotide sequence, **characterized in that** it is at least 70% identical to a nucleotide sequence according to figure 7 (SEQ ID NO 5) and/or figure 10 (SEQ ID NO 5).

5. Nucleotide sequence according to claim 4, **characterized in that** it codes for a splice variant of the p19 subunit of IL-23.

6. Amino acid sequence, **characterized in that** it is at least 70% identical to an amino acid sequence according to figure 9 (SEQ ID NO 6).

7. Amino acid sequence, **characterized in that** it forms a splice variant of the p19 subunit of IL-23.

8. Active agent especially for the treatment of diseases, **characterized in that** the active agent is a regulator according to one of claims 1 to 3.

9. Active agent especially for the treatment of diseases, **characterized in that** the active agent influences a regulator according to one of claims 1 to 3.

10. Active agent according to claim 9, **characterized in that** the active agent increases the activity of said regulator.

11. Active agent according to claim 9, **characterized in that** the active agent reduces the activity of said regulator.

12. Active agent according to one of claim 8 to 11, **characterized in that** said diseases are connected with a disturbance of IL-23 activity, wherein preferably said diseases are neurodegenerative diseases, cardiovascular diseases, immunological diseases, autoimmune diseases, inflammation diseases and/or cancer.

13. Use of an active agent according to one of claims 8 to 12 for the treatment of diseases.

14. Use of an active agent according to one of claims 8 to 12 for the manufacture of a medicament or a pharmaceutical composition for the treatment of diseases.

15. Method for the treatment of diseases, **characterized in that** at least one active agent according to one of claims 8 to 12 is administered.

16. Pharmaceutical composition, **characterized in that** it comprises at least one active agent according to one of claims 8 to 12 in an effective amount and at least one pharmaceutical carrier.

17. Use of a regulator of IL-23 for diagnosis of diseases, **characterized in that** the frequency of said regulator is analysed, wherein preferably at least one substance is used, which interacts with the regulator.

18. Use according to claim 17, **characterized in that** said regulator is a regulator according to one of claims 1 to 3.

19. Use according to claim 17 or 18, **characterized in that** said interacting substance is an antibody against the regulator.

20. Use according to one of claims 17 to 19, **characterized in that** said interacting substance is a nucleic acid molecule, which hybridises with at least a portion of a nucleic acid sequence coding for the regulator.

21. Use according to one of claims 17 to 20, **characterized in that** said diseases are connected with a disturbance of IL-23 activity, wherein preferably said diseases are neurodegenerative diseases, cardiovascular diseases, immunological diseases, autoimmune diseases, inflammation diseases and/or cancer.

22. Kit for diagnosis of diseases, comprising at least one substance, which interacts with a regulator according to one of claims 1 to 3.

23. Method for searching substances influencing the activity of IL-23, **characterized in that** a splice variant of the p19 subunit of IL-23 is used to identify and/or isolate substances interacting with the splice variant and/or IL-23.

24. Method according to claim 23, **characterized in that** the splice variant is a regulator according to one of claims 1 to 3.

25. Active agent for the treatment of diseases, **characterized in that** the active agent is identified and/or isolated by a method according to claims 23 or 24.
